# EUROPEAN PATENT APPLICATION

(11) **EP 2 899 542 A1**
(43) Date of publication of application: **29.07.2015**
(21) Application number: 13839625.4
(22) Date of filing: 19.09.2013
(51) Int. Cl.: G01N 33/579, C12M 1/34, C12Q 1/66, G01N 21/76

(54) **QUANTIFICATION METHOD, QUANTIFICATION DEVICE, AND QUANTIFICATION KIT**

(30) Priority: 20.09.2012 JP 2012207268
(71) Applicant: DKK-Toa Corporation, Shinjuku-ku Tokyo 169-8648 (JP); Hiroshima University, Higashi-Hiroshima-shi Hiroshima 739-8511 (JP)
(72) Inventor: YAWATA Satoshi, Tokyo 169-8648 (JP); HACHIYA Hiromitsu, Tokyo 169-8648 (JP); KURODA Akio, Higashihiroshima-shi Hiroshima 739-8530 (JP); NODA Kenichi, Higashihiroshima-shi Hiroshima 739-8530 (JP)
(74) Representative: Carstens, Dirk Wilhelm
(86) International application number: PCT/JP2013/075304
(87) International publication number: WO 2014/046183

(57) **Abstract**

Provided are a quantification method and a quantification device capable of performing measurement at high sensitivity and high accuracy on a sample that contains sodium ions such as a biochemical sample, a pharmaceutical product, and food.

The quantification method includes a calibration curve preparing step to measure a standard solution, which has been prepared by adding sodium ions so that a sodium ion content of the standard solution is equaled to a sodium ion content of a sample to be measured with a method employing a reaction that activates a limulus reagent and/or a biochemical luminescent reaction caused by ATP, luciferin, and luciferase, and to prepare a calibration curve that represents a relation between a measurement value and an amount of a component to be measured; a sample measuring step to measure the sample to be measured with a method being the same as that used in the calibration curve preparing step; and a quantifying step to find, by using the calibration curve, an amount of the component to be measured in the sample to be measured from a measurement value in the sample measuring step.

## Description

### TECHNICAL FIELD

The present invention relates to a quantification method, a quantification device, and a quantification kit to measure an amount of a component to be measured in a sample to be measured using a reaction that activates a limulus reagent and/or a biochemical luminescent reaction caused by ATP, luciferin or luciferin derivative (hereinafter, simply called luciferin), and luciferase or mutant luciferase (hereinafter, simply called luciferase).

### BACKGROUND ART

Conventionally, measurement of adenosine 3'-phosphate (ATP) has been performed in a variety of fields such as food sanitation, medical care, pharmaceutical products, clinical examination, and environment. ATP is contained in cells of all living beings and an ATP amount correlates with the number of cells. Accordingly, it is possible to quantify the number of cells, living microbes, and the like by measuring an ATP amount.

As a method to measure an ATP amount, there exists a biochemical luminescence method to perform measurement using a biochemical luminescent reaction caused by ATP, luciferin, and a luciferase (Patent Document 1).

To quantify living microbes or the like with the biochemical luminescence method, a luminescence amount due to a biochemical luminescent reaction is measured by an emission detector by extracting ATP in the cells using an ATP extraction reagent and causing luciferase and luciferin to act as luminescent reagents therewith. The ATP amount is measured based on correlation of the luminescence amount with the ATP amount, and then, the living microbes or the like are quantified from the ATP amount. Further, ATP amount measurement with a biochemical luminescence method has been also performed for simply measuring an ATP amount in a sample in a biochemical research field and the like.

Further, conventionally, in the fields such as medical care, pharmaceutical products, and clinical examination, measurement has been performed for an endotoxin amount that is contained in a biological sample (blood, urine, bodily fluid, tissue, extraneous matter, or another sample extracted from a living organism), a pharmaceutical product (in this specification, including a quasi-drub), or a primary material (in this specification, including a solvent, an intermediate product, and the like in addition to the primary material itself). Further, for manufacturing medical equipment (an injector, a dialysis membrane, and the like) measurement has been performed for confirming that endotoxin is not contained. When endotoxin enters a body, there may be a case that fever, shock, multi organ failure, or the like is caused. Therefore, it is important to measure and evaluate an endotoxin amount in a biological sample and to prevent endotoxin from entering a body by measuring an endotoxin amount contained in a pharmaceutical product, a primary material thereof, and the like or an endotoxin amount in a manufacturing step thereof and a manufacturing step of medical equipment.

As a method to measure an endotoxin amount, there exists a limulus test utilizing a process in which a limulus reaction system being a component of horseshoe crab amebocyte lysate (hereinafter, called a limulus reagent) is activated by endotoxin (Patent Document 2). The limulus test includes a gel-clot technique, a turbidimetric technique, a colorimetric technique, and a biochemical luminescence method having difference in the determination or measurement method.

In endotoxin amount measurement using the gel-clot technique and the turbidimetric technique, an endotoxin amount is determined or measured utilizing a reaction that a sample is gelated owing to that a limulus reagent is activated by endotoxin.

In endotoxin amount measurement using the colorimetric technique, colorimetric quantification is performed for an amount of released chromophore by measuring absorbance or a transmitted light amount using a synthetic chromogenic substrate that releases chromophore owing to that a limulus reagent is activated by endotoxin. Then, an endotoxin amount is measured based on correlation of the released chromophore amount with the endotoxin amount.

In endotoxin amount measurement with a biochemical luminescence method, released luciferin is measured based on a ATP amount measurement principle due to the abovementioned biochemical luminescent reaction using a synthetic luminescent substrate that releases luciferin owing to that a limulus reagent is activated by endotoxin. That is, a luminescence amount due to the biochemical luminescent reaction is measured by causing ATP and luciferase to act with the luciferin released by the limulus reaction system. Then, an endotoxin amount is measured based on correlation of the luminescence amount with the endotoxin amount.

Further, conventionally, in the fields such as medical care, pharmaceutical products, clinical examination, and food industry, measurement has been performed for beta-glucan. For example, it is important to measure a beta-glucan amount for selecting a treatment strategy for a patient suspected of deep mycosis, determining a treatment effect, and the like. As a method to measure a beta-glucan amount, similarly to the endotoxin amount measurement, there exists the limulus test using a gel-clot technique, a turbidimetric technique, a colorimetric technique, or a biochemical luminescence method (Patent Document 3). Each method differs from the endotoxin amount measurement in utilizing a process that the limulus reaction system is activated by beta-glucan. However, each measurement principle is approximately the same as in the case of endotoxin amount measurement.

### CITED DOCUMENT

### PATENT DOCUMENT

Patent Document 1: Japanese Patent Application Laid-Open No. H7-110301
Patent Document 2: International Laid-Open Publication No. 2009/063840
Patent Document 3: Japanese Patent Application Laid-Open No. 2010-187634

### SUMMARY OF THE INVENTION

### PROBLEM TO BE SOLVED BY THE INVENTION

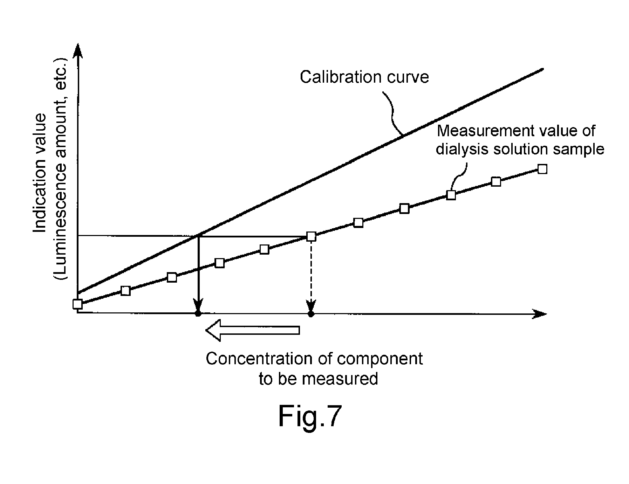

A variety of the abovementioned methods have been widely used conventionally. However, there may be a case that measurement accuracy is lowered by a disturbing component in a sample. When an amount of ATP, endotoxin, or a beta-glucan is measured with any of the abovementioned methods, for example, in a case that the sample is a dialysis solution or blood, a reaction necessary for the measurement is disturbed by sodium ions being a disturbing component in the sample. Accordingly, when quantification is performed on a component to be measured in the sample using a calibration curve prepared by using a standard solution that is prepared by adding a component to be measured (ATP, endotoxin, or beta-glucan) having a known amount (concentration) to pure water, a measurement result indicates a lower value than in reality (FIG. 7).

According to studies of the inventors, the biochemical luminescent reaction caused by ATP, luciferin, and luciferase is disturbed by sodium ions. Consequently, the measurement of an ATP amount, an endotoxin amount, and a beta-glucan amount using the biochemical luminescent reaction is disturbed by sodium ions.

Further, activation of a limulus reagent due to endotoxin and beta-glucan is disturbed by sodium ions. Consequently, the measurement of an endotoxin amount and a beta-glucan amount using the biochemical luminescent reaction is disturbed as well. Further, the measurement of an endotoxin amount and a beta-glucan amount using the limulus test due to the gel-clot technique, the turbidimetric technique, and the colorimetric technique is disturbed as well.

On such problems, there may be a case to suppress influence of a disturbing component by sufficiently diluting a sample. However, there arises a problem that sensitivity is lowered by sample diluting.

In view of the above, an object of the present invention is to provide a quantification method and a quantification device capable of performing measurement at high sensitivity and high accuracy on a sample that contains sodium ions such as a biological sample, a pharmaceutical product, and food.

Further, another object of the present invention is to provide a quantification method, a quantification device, and a quantification kit usable for the quantification device so that measurement can be performed at high sensitivity and high accuracy using the same single calibration curve on samples containing sodium ions and samples containing substantially no sodium ion or less sodium ions such as water.

### MEANS FOR SOLVING PROBLEM

The abovementioned objects are achieved with the quantification method, the quantification device, and the quantification kit of the present invention. In short, a first aspect of the present invention is a quantification method including a calibration curve preparing step to measure a standard solution, which has been prepared by adding sodium ions so that a sodium ion content of the standard solution is equaled to a sodium ion content of a sample to be measured with a method employing a reaction that activates a limulus reagent and/or a biochemical luminescent reaction caused by ATP, luciferin, and luciferase, and to prepare a calibration curve that represents a relation between a measurement value and an amount of a component to be measured; a sample measuring step to measure the sample to be measured with a method being the same as that used in the calibration curve preparing step; and a quantifying step to find, by using the calibration curve, an amount of the component to be measured in the sample to be measured from a measurement value in the sample measuring step.

A second aspect of the present invention is a quantification device performing a calibration curve preparing step to measure a standard solution, which has been prepared by adding sodium ions so that a sodium ion content of the standard solution is equaled to a sodium ion content of a sample to be measured with a method employing a reaction that activates a limulus reagent and/or a biochemical luminescent reaction caused by ATP, luciferin, and luciferase, and to prepare a calibration curve that represents a relation between a measurement value and an amount of a component to be measured; a sample measuring step to measure the sample to be measured with a method being the same as that used in the calibration curve preparing step; and a quantifying step to find, by using the calibration curve, an amount of the component to be measured in the sample to be measured from a measurement value in the sample measuring step.

A third aspect of the present invention is a quantification kit for quantifying a component to be measured in a sample to be measured with a method employing a reaction that activates a limulus reagent and/or a biochemical luminescent reaction caused by ATP, luciferin, and luciferase or for preparing a calibration curve to be used for the quantifying, the quantification kit including a sodium ion source that supplies a predetermined amount of sodium ions to the sample to be measured or a standard solution for preparing the calibration curve.

### EFFECT OF THE INVENTION

According to the present invention, measurement can be performed at high sensitivity and high accuracy on a sample that contains sodium ions such as a biological sample, a pharmaceutical product, and food. Further, according to the present invention, measurement can be performed at high sensitivity and high accuracy using the same calibration curve on samples containing sodium ions and samples containing substantially not sodium ion or less sodium ions such as water.

### BRIEF DESCRIPTION OF THE DRAWINGS

FIG.1 is a schematic functional block diagram of a quantification device according to an embodiment of the present invention.
FIG. 2 is a schematic view for explaining an example of a quantification kit according to the present invention.
FIG. 3 is a graph indicating influence of sodium ions to measurement of an ATP amount with a biochemical luminescence method and an effect to suppress the influence.
FIG. 4 is a graph indicating influence of sodium ions to measurement of an endotoxin amount with the biochemical luminescence method and an effect to suppress the influence.
FIG. 5 is a graph indicating influence of sodium ions to measurement of an endotoxin amount using a limulus test with a turbidimetric technique and an effect to suppress the influence.
FIG. 6 is a graph indicating a relation between deviation of an NaCl concentration of a standard solution and a measurement result of an endotoxin amount.
FIG. 7 is an explanatory view for explaining a problem in the related art.

### EMBODIMENTS OF THE INVENTION

In the following, a quantification method, a quantification device, and a quantification kit according to the present invention will be described in detail with reference to the drawings.

### 1. Quantification method

The quantification method according to the present invention includes the following steps.
(a) A calibration curve preparing step for measuring a standard solution, which has been prepared by adding sodium ions so that a sodium ion content thereof is equivalent to a sodium ion content of a sample to be measured with a method employing a reaction that activates a limulus reagent and/or a biochemical luminescent reaction caused by ATP, luciferin, and luciferase, and for preparing a calibration curve that represents a relation between a measurement value and an amount of a component to be measured.
(b) A sample measuring step for measuring the sample to be measured with a method being the same as that used in the calibration curve preparing step.
(c) A quantifying step for finding, by using the calibration curve, an amount of the component to be measured in the sample to be measured from a measurement value obtained in the sample measuring step.

The component to be measured varies in accordance with the method used in the calibration curve preparing step (a) and the sample measuring step (b). When the measurement is performed with the method employing the reaction that activates a limulus reagent, it is possible to adopt endotoxin or beta-glucan. When the measurement is performed with the method employing the biochemical luminescent reaction caused by ATP, luciferin, and luciferase, it is possible to adopt ATP. Further, when the measurement is performed with the method employing the reaction that activates a limulus reagent and the biochemical luminescent reaction caused by ATP, luciferin, and luciferase, it is possible to adopt endotoxin or beta-glucan.

Here, in the case that ATP is adopted as the component to be measured, it is possible to include a case that ATP is measured as extracting ATP from viable cells or the like as having a final target as quantifying viable cells or the like in the sample to be measured. In this case, it is possible to find a viable cell count using a calibration curve that represents a relation between an ATP amount and a viable cell count being the final quantification target after the ATP amount is measured through the abovementioned steps. Alternatively, it is also possible to find a viable cell count by preparing a relational expression between a luminescence amount and a viable cell count from the calibration curve obtained in step (a) and the calibration curve representing the relation between an ATP amount and a viable cell count. Here, ATP in cells such as viable cells can be extracted by using an ATP extraction reagent.

The measurement described above is based on the following principles. Here, since the measurement method is well known, detail description will be skipped.

(1) Measurement of an endotoxin amount with the method employing the reaction that activates a limulus reagent (hereinafter, also called endotoxin amount measurement using a limulus reagent)
   A Factor-C-based limulus reaction system being a chain reaction system which is started by endotoxin exists in horseshoe crab amebocyte lysate (limulus amebocyte lysate (LAL)) contained in the limulus reagent. Endotoxin activates Factor C. The activated Factor C activates Factor B of the limulus reaction system. The activated Factor B activates a clotting enzyme precursor of the limulus reaction system and generates a clotting enzyme. Gelation occurs by the action of the generated clotting enzyme.

The measurement of an endotoxin amount with a limulus reagent to be performed using the abovementioned processes can be performed by measuring a state of a gelated reagent (i.e., a gel-clot technique) or measuring turbidity (i.e., a turbidimetric technique). Alternatively, it is also possible to measure an endotoxin amount by adding a synthetic chromogenic substrate to the abovementioned reaction system to release chromophore and measuring an amount thereof based on absorbance or a transmitted light amount (i.e., a colorimetric technique).

(2) Measurement of a beta-glucan amount with the method employing the reaction that activates a limulus reagent (hereinafter, also called beta-glucan amount measurement using a limulus reagent)

A Factor-G-based limulus reaction system being a chain reaction system which is started by beta-glucan exists in horseshoe crab amebocyte lysate contained in the limulus reagent. Beta-glucan activates Factor G. The activated Factor G activates a clotting enzyme precursor of the limulus reaction system and generates a clotting enzyme. Gelation occurs by the action of the generated clotting enzyme.

Similarly to the measurement of an endotoxin amount, the measurement of beta-glucan amount with a limulus reagent to be performed using the abovementioned processes can be performed by the gel-clot technique, the turbidimetric technique, or the colorimetric technique.

(3) Measurement of an ATP amount with the method of employing a biochemical luminescent reaction caused by ATP, luciferin, and luciferase (hereinafter, called ATP amount measurement with a biochemical luminescence method)

ATP reacts with luciferin by the action of luciferase in the presence of Mg2+ (divalent metallic ions) and generates AMP, oxyluciferin, and pyrophosphoric acid. Since a luminescence amount of light generated at that time is correlated with an ATP amount, the ATP amount can be measured based thereon.

(4) Measurement of an endotoxin amount with the method employing the reaction that activates a limulus reagent and a biochemical luminescent reaction caused by ATP, luciferin, and luciferase (hereinafter, also called endotoxin amount measurement with a biochemical luminescence method)

The endotoxin amount measurement with the biochemical luminescence method is performed by applying the ATP amount measurement with the biochemical luminescence method described as (3) to the processes of the limulus reaction system described as (1). That is, a synthetic luminescent substrate that contains luciferin being a luminescent substrate is added to the limulus reaction system to release luciferin with activation of the limulus reaction system due to endotoxin. A luminescence amount due to the biochemical luminescent reaction is measured by causing ATP and luciferase to act with the released luciferin. Since the luminescence amount is correlated with the released luciferin amount (i.e., a degree of activation of the limulus reaction system), it is possible to measure endotoxin amount as a result.

(5) Measurement of a beta-glucan amount with the method employing the reaction that activates a limulus reagent and a biochemical luminescent reaction caused by ATP, luciferin, and luciferase (hereinafter, also called beta-glucan amount measurement with a biochemical luminescence method)

Similarly to (4), the beta-glucan amount measurement with the biochemical luminescence method is performed by applying the ATP amount measurement with the biochemical luminescence method to the processes of the limulus reaction system. That is, a synthetic luminescent substrate that contains luciferin is added to the limulus reaction system to release luciferin with activation of the limulus reaction system due to beta-glucan. Then, a beta-glucan amount is measured by measuring a luminescence amount due to the biochemical luminescent reaction.

As described above, the biochemical luminescent reaction caused by ATP, luciferin, and luciferase is inhibited by sodium ions. Accordingly, measurement of an ATP amount, an endotoxin amount, and a beta-glucan amount with the biochemical luminescence method is inhibited by sodium ions.

Further, the activation of the limulus reaction system due to endotoxin and the beta-glucan is inhibited by sodium ions. Accordingly, measurement of an endotoxin amount and a beta-glucan amount using a limulus reagent and measurement of an endotoxin amount and a beta-glucan amount with the biochemical luminescence method are inhibited.

For example, in the case that measurement of an ATP amount, an endotoxin amount, or a beta-glucan amount with the biochemical luminescence method is performed on a sample that contains sodium ions in relatively high concentrations, such as a dialysis solution and blood, a measurement result indicates a lower value than in reality (see FIG. 7) when a component to be measured in the sample is quantified with a calibration curve prepared by using a standard solution that is prepared by adding a component to be measured (ATP, endotoxin, or beta-glucan) having a known amount (concentration) to a solvent such as pure water without containing a sodium ion.

In view of the above, according to the quantification method of the present invention, in the calibration curve preparing step of (a), measurement is performed on a standard solution to which sodium ions are added so that a sodium ion content thereof is equaled to a sodium ion content of a sample to be measured and a calibration curve is prepared based on the measurement value. That is, in advance of quantification of a sample that contains a large volume of a disturbing component (sodium ions) such as a dialysis solution and blood, the calibration curve is prepared based on the measurement result of the standard solution to which the disturbing component is added thereto so that the disturbing component content thereof equals to the disturbing component content contained in the sample to be measured, and then, quantification of the component to be measured in the sample is performed using the calibration curve. Thus, influence of the disturbing component is to be cancelled.

In the calibration curve preparing step of (a), standard solutions are prepared over a range of desired contents (concentrations) with desired content (concentration) increments in accordance with an amount of a component to be measured in the sample to be measured so that desired measurement accuracy can be obtained. Here, it is preferable that measurement in the calibration curve preparing step of (a) is performed under substantially the same conditions (contents (concentrations) of components of the reaction system excluding the component to be measured, reaction temperature, reaction time, and the like) as for measurement in the sample measurement step of (b). Under equaled conditions, it is possible to perform the measurement at higher sensitivity and higher accuracy. The sample to be measured may be an injectable solution, a medical agent to be used in medical practice such as an infusion solution and a dialysis solution, an external medicine such as eye-drops, a pharmaceutical product such as a variety of internal medicines, water (common water, RO water, purified water, disinfected-purified water, sterilized water, injectable water (injectable distillated water), pure water, ultrapure water, reverse osmosis water, and the like), collected matters from medical equipment and medical devices, collected matters from a clean room, a biological sample (clinical sample) such as blood (whole blood, blood serum, blood plasma) and urine, or the like. Further, a variety of samples in food processing, food sanitation, and environmental fields can be used as a sample to be measured.

Here, the content being equal to the sodium ion content in the sample to be measured denotes to be equal in the order that the influence of sodium ions to the measurement result can be suppressed in accordance with the desired measurement accuracy. As described later in detail, according to the study of the inventors, the influence of sodium ions to the measurement result can be suppressed when sodium content of the standard solution is within a range of -50% to +75% of that of the sample to be measured. It is preferable to be in a range of -15% to +25%, and more preferable to be in a range of -5% to +10%.

For performing measurement at high sensitivity and high accuracy with the same calibration curve, it is required for respective samples such as a dialysis solution and blood containing sodium ions that the sodium ion contents are equaled. In such a case as well, the sodium ion contents being equal to each other denotes the same as the above. As long as the sodium ion contents are equaled, the measurement can be performed at high sensitivity and high accuracy with the same calibration curve even for different kinds of samples such as a dialysis solution and blood, for example.

Further, in the calibration curve preparing step of (a), it is preferable that sodium ions are added to the standard solution or the sample to be measured by adding sodium chloride (NaCl). Here, adding sodium ions to the standard solution or the sample to be measured is not limited to pouring or injecting a sodium ion source such as NaCl to the standard solution or the sample as a dry agent or a solution. For example, it is also possible to adopt a method to pour the solution or the sample to be measured into a container that accommodates a sodium ion source such as NaCl as a dry agent or a solution. Further, in the case that another reagent (e.g., a buffer solution, luciferin, luciferase, ATP, divalent metallic ions, a synthetic luminescent substrate, a synthetic chromogenic substrate, or the like) to be poured or injected to the standard solution or the sample to be measured (or diluted solutions thereof) contains a sodium ion source such as NaCl, it is also possible to adopt a method to add sodium ions to the standard solution or the sample to be measured so that a sodium ion amount thereof becomes to a predetermined value by pouring or injecting the reagent thereto.

### 2. Quantification device

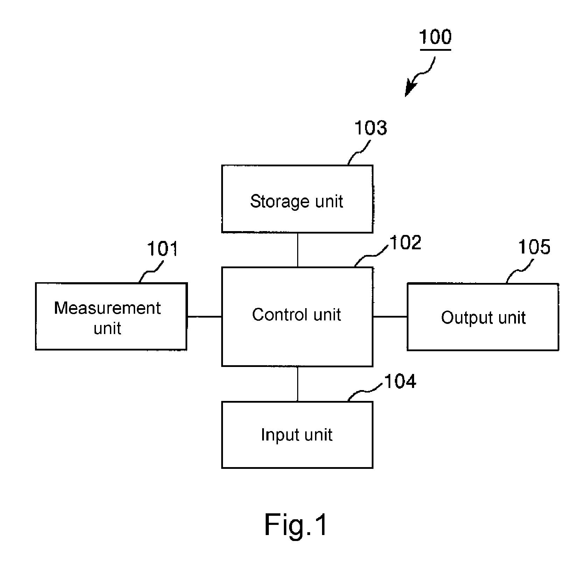

The quantification device according to the present invention includes the abovementioned steps of (a), (b), and (c). FIG. 1 illustrates a schematic functional block of an embodiment of the quantification device according to the present invention.

As illustrated in FIG. 1, a quantification device 100 includes a measurement unit 101 to perform measurement, a control unit 102 to perform control of measurement operations, a storage unit 103 to store information, an input unit 104 to input information to the control unit 102, an output unit 105 to output information from the control unit 102, and the like.

The measurement unit 101 is structured in accordance with the method to be adopted in the calibration curve preparing step of (a) and the sample measuring step of (b) described above. For example, in the case of adopting the method for measuring an ATP amount, an endotoxin amount, or a beta-glucan amount with the biochemical luminescence method, the measurement unit 101 may be structured with a reaction container, an emission detector, a supply unit of a solution to be detected (a sample to be measured, a standard solution), a reagent supply unit, and the like. In the case of adopting the method for measuring an endotoxin amount or a beta-glucan amount using a limulus reagent, for example, when the turbidimetric technique or colorimetric technique is adopted, the measurement unit 101 may be structured with a reaction container, an absorbance photometer (transmitted light detector), a supply unit of a solution to be detected, a reagent supply unit, and the like.

The control unit 102 may totally control operations of the quantification device 100. The control unit 102 may be structured with a microcomputer or the like, in accordance with instruction information from the input unit 104, to perform sequence control of the measurement unit 101 in accordance with programs and various kinds of setting information stored in the storage unit 103, to process measurement results measured by the measurement unit 101, and to output information of the measurement results to the output unit 105.

The storage unit 103 is structured with an electronic memory or the like so as to store a variety of information such as control program and various kinds of setting information to be used by the control unit 102, measurement result information acquired by the control unit 102, and the like.

The input unit 104 is structured with an operation unit and the like having input keys and the like arranged at the quantification device 100 for inputting, to the control unit 102, a variety of information such as various kinds of setting information, instruction information of start/stop of measurement, and the like. Alternatively, the input unit 104 may be an interface unit that receives information being similar to the above from equipment arranged outside the quantification device 100 and transmits the information to the control unit 102.

The output unit 105 may be structured with a liquid crystal display or the like to display various kinds of setting information and measurement result information, a printer unit to print out such information, or the like. Alternatively, the output unit 105 may be an interface unit that receives information being similar to the above from the control unit 102 and transmits to equipment arranged outside the quantification device 100.

Next, description will be provided on a case to quantify endotoxin contained in a dialysis solution and a solution for preparing a dialysis solution (RO water for diluting undiluted solution) using the quantification device according to an embodiment of the present invention.

### (Calibration curve preparing step)

Prior to quantifying endotoxin (a component to be measured) in the dialysis solution and the RO water (sample to be measured), a calibration curve is prepared in a manner described below.

Sodium ions are added to each of two or more standard solutions that contain different endotoxin amounts so that the sodium ion contents of the standard solutions are equaled to the sodium ion content of a dialysis solution. Specifically, since the sodium ion contained in the dialysis solution is about 140 mmol/L, sodium ions are added so that the sodium ion concentrations of the respective standard solutions become to 140 mmol/L.

Next, measurement is performed on the respective standard solutions with endotoxin amount measurement using a limulus reagent or endotoxin amount measurement with a biochemical luminescence method and a calibration curve is prepared.

At that time, the control unit 102 causes the measurement unit 101 to perform the abovementioned measurement on the standard solutions in accordance with control programs and various kinds of setting information stored in the storage unit 103 and to prepare the calibration curve from the obtained measurement result, and then, causes the storage unit 103 to store the calibration curve. The calibration curve is used for quantification of a component to be measured in a sample to be measured.

### (Sample measuring step)

Measurement is performed on the dialysis solution and the RO water respectively while the method and conditions of the measurement are equaled to those in the abovementioned calibration curve preparing step.

Here, in the case that measurement is performed on the dialysis solution that is taken as the basis for the sodium ion content of the standard solutions in the calibration curve preparing step, sodium ions are not added thereto. In contrast, in the case that measurement is performed on the RO water having a less sodium ion content than that in the dialysis solution, measurement is performed after sodium ions are added so that the sodium ion concentration becomes to 140 mmol/L as in the calibration curve preparing step.

At that time, the control unit 102 causes the abovementioned measurement to be performed on the sample to be measured in accordance with control programs and various kinds of setting information stored in the storage unit 103. The information of the measurement result obtained by the measurement unit 101 is stored in the storage unit 103 and used for quantification of a component to be measured in a sample to be measured.

### (Quantifying step)

Both the measurement value of the dialysis solution and the measurement value of the RO water measured in the sample measuring step are converted into endotoxin amounts using the single calibration curve that is prepared in the abovementioned calibration curve preparing step.

Owing to performing quantification with the steps described above, measurement can be performed on the dialysis solution at high sensitivity and high accuracy without being influenced by sodium ions being a disturbing component. Further, accurate measurement can be performed on the RO water that contains few sodium ions using the same single calibration curve.

For example, it is possible with inputting of an operator to provide, to the device, information whether or not measurement of a sample to be measured is performed after sodium ions are added thereto. Alternatively, it is also possible to set, as an operational sequence, an introduction order of a plurality of samples to be measured and necessity of adding sodium ions. Instead, it is also possible to automatically decide necessity of adding sodium ions and an amount of sodium ions to be added in accordance with a measurement value of a sodium ion measuring unit (e.g., an ion electrode or a permittivity meter) arranged in the quantification device or in accordance with a signal from the outside (e.g., a signal from an ion concentration meter or a permittivity meter arranged separately from the quantification device).

Here, samples to be measured are not limited to two kinds. It is possible that three or more kinds of samples can be measured while adding of sodium ions is controlled. Following are examples, in a pharmaceutical product manufacturing process, for measuring three kinds of samples to be measured being a finished product, an intermediate product, and a primary material. When sodium ion contents thereof are arranged in the order of the intermediate product, the finished product, and the primary material in descending order, it is possible to prepare a calibration curve of the calibration curve preparing step as taking the sodium ion content of the intermediate product as the basis therefor and to perform measurement on the finished product and the primary material with sodium ions added so that the sodium ion contents thereof are equaled to that of the intermediate product. Alternatively, it is also possible to prepare a calibration curve of the calibration curve preparing step as taking the sodium ion content of the finished product as the basis therefor and to perform measurement on the finished product and the intermediate product without adding sodium ions and on the primary material with sodium ions added so that the sodium ion content thereof is equaled to that of the finished product.

As described above, samples to be measured may be a plurality of samples having different sodium ion contents. In this case, it is possible, in the calibration curve preparing step, to prepare a calibration curve by performing measurement on a standard solution to which sodium ions are added so that the sodium ion content thereof is equaled to that of a given sample among the plurality of samples. Then, in the sample measuring step, measurement is performed without adding sodium ions on the given sample and a sample that has a sodium ion content being equal to or greater than that of the given sample. On the other hand, regarding a sample that has a sodium ion content being less than that of the given sample, measurement is performed on the sample after sodium ions are added so that the sodium ion content is equaled to that of the given sample.

### 3. Quantification kit

Description will be provided on a quantification kit of the present invention.

The quantification kit of the present invention can be used preferably for the quantification method and the quantification device described above. That is, the quantification kit includes a sodium ion source that supplies a predetermined amount of sodium ions to a sample to be measured or a standard solution for preparing a calibration curve when a component to be measured in the sample to be measured is to be quantified with the method employing the reaction that activates a limulus reagent and/or the biochemical luminescent reaction caused by ATP, luciferin, and luciferase or when the calibration curve used for the quantification is to be prepared.

For example, in the case of measuring an endotoxin amount in RO water for diluting undiluted solution in addition to measuring an endotoxin amount in a dialysis solution as described above, the quantification kit is configured to include a sodium ion source for supplying sodium ions to the standard solution and the RO water so that the sodium ion contents thereof are equaled to that of the dialysis solution for enabling the measurement to be performed on the dialysis solution and the RO water using the same single calibration curve.

The sodium ion concentration of the dialysis solution is about 140 mmol/L while the standard solution and the RO water contain few sodium ions. When amounts of the standard solution and the RO water to be used for the measurement using the quantification kit are kept constant continuously, a predetermined amount of sodium ions to be supplied by the quantification kit can be constant as well and the amount can be easily calculated. That is, the sodium ion supply amount of the quantification kit can be appropriately determined in accordance with a sodium ion concentration of the sample to be measured and a liquid amount to be used for the measurement with the quantification kit.

The quantification kit of the present invention can be preferably used especially in the case that the sample to be measured is a biological sample such as blood and in the case that the sample is an injectable solution, an infusion solution, a dialysis solution, eye-drops, a normal saline solution, or the like. Since the sodium ion concentrations thereof are approximately constant, the quantification kit capable of supplying sodium ions by the amount acquired from the concentration can be used with mass-production.

Specifically, the sodium ion concentration of blood is in a range of 135 to 145 mmol/L, the sodium ion concentration of a dialysis solution is about 140 mmol/L, and the sodium ion concentration of normal saline solution is about 155 mmol/L. The quantification kit is configured to be capable of supplying sodium ions by the amount calculated based on the above. Here, it is preferable that the sodium ion source of the quantification kit supplies sodium ions so that the sodium ion concentration of the sample to be measured or the standard solution for preparing the calibration curve is to be in a range of 135 to 155 mmol/L.

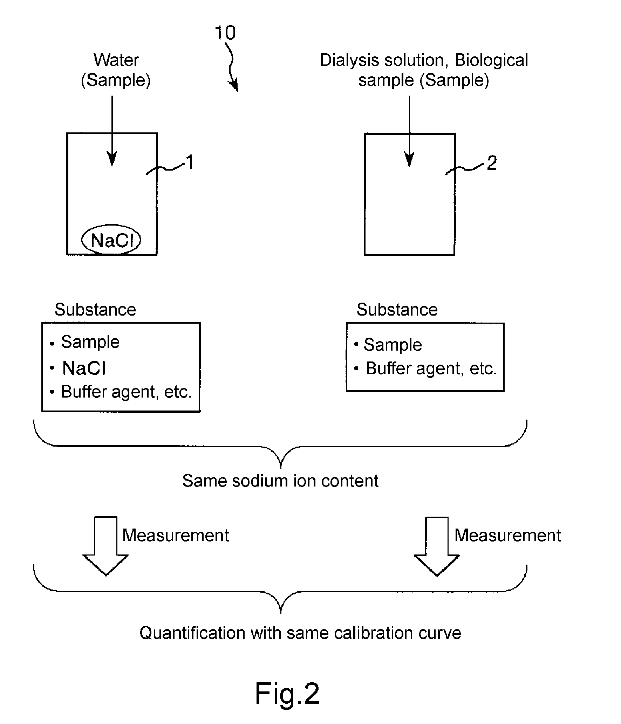

More specifically, as illustrated in FIG. 2 for example, a quantification kit 10 may include a first container 1, as a sodium ion source, that accommodates NaCl as a dry agent or a solution. Further, the first container 1 may accommodate a pH buffer agent as a dry agent or a solution (buffer solution). Then, a standard solution or a sample such as water that contains few or less sodium ions can be injected into the first container 1.

Further, the quantification kit 10 may include a second container 2 in which a sodium ion source is not accommodated. The second container 2 may accommodate a pH buffer agent as a dry agent or a solution (buffer solution). Then, a sample such as a dialysis solution that contains sodium ions can be injected into the second container 2.

Sodium ion concentrations of substance in the first container 1 and the second container 2 into which the sample or the standard solution is injected are set equaled. Then, measurement is performed using the sample or the standard solution in the first container 1 and the second container 2.

### [Examples]

Next, the present invention will be further described by way of specific examples.

### [Example 1]

Examining was performed on the influence of sodium ions to measurement of an ATP amount with the biochemical luminescence method and an effect to suppress the influence.

In the present example, ATP-containing solutions including ATP diluted into different concentrations were prepared using water, a dialysis solution, and an aqueous NaCl solution respectively as a solvent. Then, a relation between the ATP concentration and a luminescence amount due to a biochemical luminescent reaction was obtained for each of the ATP-containing solutions.

### <Reagent>

AF-2A1 manufactured by DKK-TOA Corporation was used as the ATP standard solution. In brief, the ATP standard solution was prepared by adding a solution of 1×10⁻⁷ M of ATP to 0.025 M of HEPES buffer solution. Further, injection water (Otsuka Distilled Water manufactured by Otsuka Pharmaceutical Co., Ltd.) was used as water. A luminescent reagent containing luciferin and mutant luciferase (Luciferase FM+ manufactured by Bioenex) was used as luciferin and mutant luciferase. Kindary 3D (manufactured by Fuso Pharmaceutical Industries Ltd.) was used as a dialysis agent. In Kindary 3D, an A-agent of a dialysis solution is structured with an A-1 agent being an electrolyte component and an A-2 agent being a glucose (non-electrolyte) component.

### <Method>

The dialysis solution was prepared by dissolving the A-agent and a B-agent of Kindary 3D with injection water in accordance with prescription. The sodium ion concentration of Kindary 3D is 140 mmol/L (140 mEq/L).

The aqueous NaCl solution was prepared to have a concentration being 140 mmol/L (140 mEq/L) by dissolving NaCl with injection water. Thus, the sodium ion concentration of the aqueous NaCl solution was the same as that of the dialysis solution.

The ATP-containing solutions were prepared to have concentrations being 1×10⁻¹⁴, 1×10⁻¹³, 1×10⁻¹², 1×10⁻¹¹, 1×10⁻¹⁰, 1×10⁻⁹, and 1×10⁻⁸, respectively by diluting the abovementioned ATP standard solution with injection water, the dialysis solution, and the aqueous NaCl solution.

Next, 100 µL of each ATP-containing solution was filled into each reaction container, and then, 100 µL of a luminescent reagent was added into each reaction container and mixed therein. Subsequently, luminescence amounts of the reaction solutions were measured using an emission detector (faint emission detector manufactured by Hamamatsu Photonics K.K.).

### <Results>

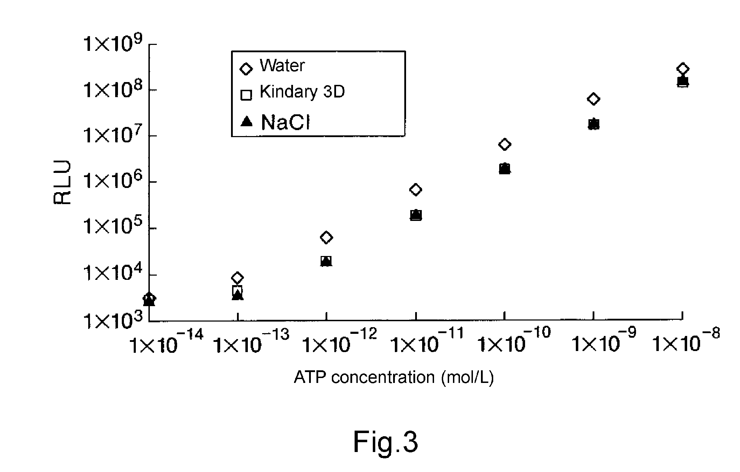

FIG. 3 shows the results. The horizontal axis of FIG. 3 represents an ATP concentration (mol/L) of the ATP-containing solutions and the vertical axis thereof represents a luminescence amount (luminescence strength: RLU).

In the case of using water as a solvent, the relation between the ATP concentration and the luminescence amount largely differs from that in the case of using a dialysis solution as a solvent. In particular, as illustrated in FIG. 3, luminescence amounts at the respective ATP concentrations are lower when a dialysis solution was used as a solvent than those when water was used as a solvent. Therefore, when ATP in a dialysis solution is quantified using a calibration curve that is prepared with a standard solution prepared by simply adding ATP having a known concentration to water, a measurement result having a lower concentration than in reality is obtained.

In contrast, in the case of using an aqueous NaCl solution as a solvent, the relation between the ATP concentration and the luminescence amount is approximately matched with that in the case of using a dialysis solution as a solvent. Therefore, owing to using a calibration curve that is prepared with a standard solution prepared by adding ATP having a known concentration to an aqueous NaCl solution containing sodium ions at the same concentration as the dialysis solution, it turns out that the ATP amount in the dialysis solution can be measured at high accuracy. Further, since the sample is not required to be diluted to suppress the influence of sodium ions, it turns out that measurement can be performed at high sensitivity. Furthermore, in the case that the sample is, for example, water without containing a sodium ion, it turns out that measurement can be performed at high sensitivity and high accuracy even for ATP in the sample using the same calibration curve as the case of the dialysis solution by adding NaCl thereto so as to contain sodium ions at the same concentration as that of the standard solution (i.e., as that of the dialysis solution).

### [Example 2]

Examining was performed on the influence of sodium ions to measurement of an endotoxin amount with the biochemical luminescence method and an effect to suppress the influence.

In the present example, endotoxin-containing solutions including endotoxin diluted into different contents were prepared using water, a dialysis solution, and an aqueous NaCl solution respectively as a solvent. Then, a relation between the endotoxin amount and a luminescence amount due to a biochemical luminescent reaction was obtained for each of the endotoxin-containing solutions.

### <Reagent>

Limulus ES-II (manufactured by Wako Pure Chemical Industries, Ltd.) being an endotoxin measurement reagent was used as the limulus reagent (LAL). Control Standard Endotoxin (CSE) (manufactured by Wako Pure Chemical Industries, Ltd.) was used as an endotoxin standard substance. Injection water (Injection Water manufactured by Otsuka Pharmaceutical Co., Ltd.) was used as water. Benzoil-Leu-Gly-Arg-luciferin was used as a synthetic luminescent substrate. Mutant luciferase (luciferase FM manufactured by Bioenex) was used as luciferase. Kindary 3D (manufactured by Fuso Pharmaceutical Industries Ltd.) and Carbostar P (manufactured by Ajinomoto Pharmaceuticals Co., Ltd.) were used as a dialysis agent. Carbostar P is an A-agent of a dialysis solution in a form of a single agent.

### <Method>

The dialysis solution was prepared by dissolving each of Kindary 3D and Carbostar P with injection water in accordance with prescription. The sodium ion concentration of each solution of Kindary 3D and Carbostar P is 140 mmol/L (140 mEq/L).

The aqueous NaCl solution was prepared to have a concentration being 140 mmol/L (140 mEq/L) by dissolving NaCl with injection water. Thus, the sodium ion concentration of the aqueous NaCl solution was the same as that of the dialysis solution.

The endotoxin-containing solutions were prepared to have contents being 0.001, 0.01, 0.1, and 1 EU/mL, respectively by diluting an undiluted solution with injection water, the dialysis solution, and the aqueous NaCl solution. Here, the undiluted solution was prepared by dissolving the endotoxin standard substance with injection water to have a content being 1000 EU/mL.

The limulus reagent was prepared using 200 µL of injection water.

Next, 50 µL of each endotoxin-containing solution was filled into each reaction container, 25 µL of the limulus reagent was added into each reaction container and mixed therein, and then, heating was performed for 20 minutes at 37 °C.

Next, 50 µL of the synthetic luminescent substrate of 6.7×10⁻⁵ M dissolved into tricine buffer solution (pH 8.5) of 0.04 M containing magnesium acetate of 0.001 M and 5% trehalose was added into each reaction container, and then, heating was performed for five minutes at 37 °C.

Next, 50 µL of ATP of 10 M dissolved into a HEPES buffer solution (pH 7) of 0.025 M and 50 µL of luciferase dissolved into tricine buffer solution (pH 8.5) of 0.04 M containing magnesium acetate of 0.001 M and trehalose of 0.15 M (one gram of FM is dissolved in 4 mL) were added to each reaction container. Subsequently, luminescence amounts of the reaction solutions were measured using an emission detector (AF-100 manufactured by DKK-TOA Corporation).

### <Results>

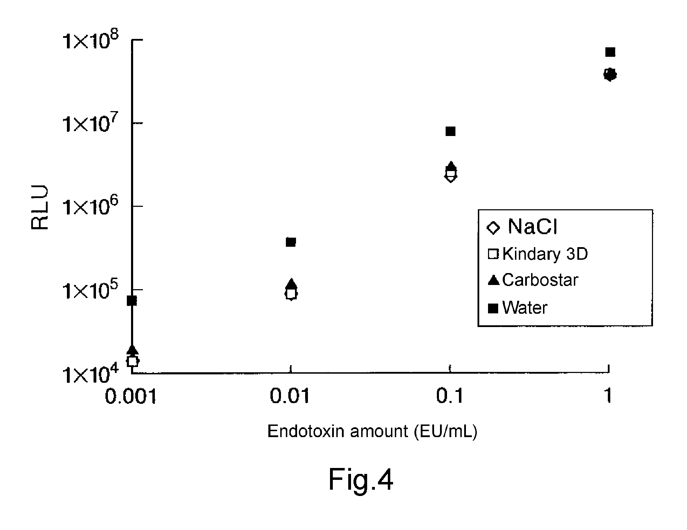

FIG. 4 shows the results. The horizontal axis of FIG. 4 represents an endotoxin amount (EU/L) of the endotoxin-containing solutions and the vertical axis thereof represents a luminescence amount (luminescence strength: RLU).

In the case of using water as a solvent, the relation between the endotoxin amount and the luminescence amount largely differs from that in the case of using a dialysis solution as a solvent. In particular, as illustrated in FIG. 4, luminescence amount at the respective endotoxin amounts are lower when a dialysis solution was used as a solvent than those when water was used as a solvent. Therefore, when endotoxin in a dialysis solution is quantified using a calibration curve that is prepared with a standard solution prepared by simply adding endotoxin having a known amount to water, a measurement result having a lower amount than in reality is obtained.

In contrast, in the case of using an aqueous NaCl solution as a solvent, the relation between the endotoxin concentration and the luminescence amount is approximately matched with that in the case of using a dialysis solution as a solvent. Therefore, owing to using a calibration curve that is prepared with a standard solution prepared by adding endotoxin having a known amount to an aqueous NaCl solution containing sodium ions at the same concentration as the dialysis solution, it turns out that the endotoxin amount in the dialysis solution can be measured at high accuracy. Further, since the sample is not required to be diluted to suppress the influence of sodium ions, it turns out that measurement can be performed at high sensitivity. Furthermore, in the case that the sample is, for example water without containing a sodium ion, it turns out that measurement can be performed at high sensitivity and high accuracy even for endotoxin in the sample using the same calibration curve as the case of the dialysis solution by adding NaCl thereto so as to contain sodium ions at the same concentration as that of the standard solution (i.e., as that of the dialysis solution).

### [Example 3]

Examining was performed on the influence of sodium ions to measurement of an endotoxin amount using a limulus test with the turbidimetric technique and an effect to suppress the influence.

In the present example, endotoxin-containing solutions including endotoxin diluted into different contents were prepared using water, a dialysis solution, and an aqueous NaCl solution respectively as a solvent. Then, a relation between the endotoxin amount and a measurement value of a reaction time due to the turbidimetric technique was obtained for each of the endotoxin-containing solutions.

### <Reagent>

The same as in example 2 was used as the limulus reagent (LAL) and the endotoxin standard substance. The same as in example 2 was used as water. Further, Kindary 3D being the same as in example 1 was used as the dialysis agent.

<Method>

The dialysis solutions and the aqueous NaCl solution were prepared similarly to example 1 and example 2.

The endotoxin-containing solutions were prepared to have contents being 0.00125, 0.0025, 0.005, and 0.01 EU/mL, respectively by diluting an undiluted solution with injection water, the dialysis solution, and the aqueous NaCl solution. Here, the undiluted solution was prepared by dissolving the endotoxin standard substance with injection water to have a content being 1000 EU/mL.

Next, 200 µL of each endotoxin-containing solution was filled into each reaction container containing the limulus reagent. Subsequently, a reaction time of each reaction solution until the transmitted light amount was reduced to a predetermined threshold value (about 90%) was measured using measurement equipment (ET-6000 manufactured by Wako Pure Chemical Industries, Ltd.).

### <Results>

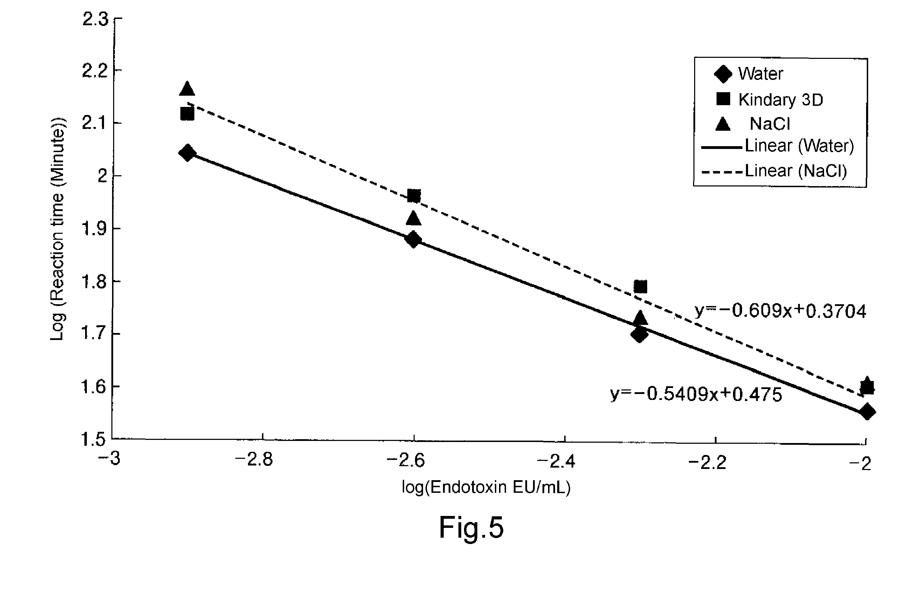

FIG. 5 shows the results. The horizontal axis of FIG. 5 represents an endotoxin amount (log (EU/mL)) of the endotoxin-containing solutions and the vertical axis thereof represents a reaction time (log (minute)).

In the case of using water as a solvent, the relation between the endotoxin amount and the reaction time largely differs from that in the case of using a dialysis solution as a solvent. In particular, as illustrated in FIG. 5, reaction times at the respective endotoxin amounts are longer when a dialysis solution was used as a solvent than those when water was used as a solvent. Therefore, when endotoxin in a dialysis solution is quantified using a calibration curve that is prepared with a standard solution prepared by simply adding endotoxin having a known amount to water, a measurement result having a lower amount than in reality is obtained.

In contrast, in the case of using an aqueous NaCl solution as a solvent, the relation between the endotoxin amount and the reaction time becomes close to the case of using a dialysis solution as a solvent. Therefore, owing to using a calibration curve that is prepared with a standard solution prepared by adding endotoxin having a known amount to an aqueous NaCl solution containing sodium ions at the same concentration as the dialysis solution, it turns out that the endotoxin amount in the dialysis solution can be measured at high accuracy. Further, since the sample is not required to be diluted to suppress the influence of sodium ions, it turns out that measurement can be performed at high sensitivity. Furthermore, in the case that the sample is, for example water without containing a sodium ion, it turns out that measurement can be performed at high sensitivity and high accuracy even for endotoxin in the sample using the same calibration curve as the case of the dialysis solution by adding NaCl thereto so as to contain sodium ions at the same concentration as that of the standard solution (i.e., as that of the dialysis solution).

Table 1 indicates calculation results of recovery, for samples with a known amount of endotoxin added to a dialysis solution, in the case of using, as the calibration curve illustrated in FIG. 5, the relation where water was used as a solvent (water calibration curve) and the relation where an aqueous NaCl solution was used as a solvent (NaCl) after measuring reaction times with the turbidimetric technique in accordance with the abovementioned procedure.

**[Table 1]**

| | Recovery | |
|---|---|---|
| Endotoxin amount (EU/mL) | Calculated using water calibration curve | Calculated using NaCl calibration curve |
| 0.00125 | 73.6 | 108.3 |
| 0.0025 | 69.3 | 95.0 |
| 0.005 | 70.8 | 89.6 |
| 0.01 | 79.5 | 91.9 |

When the endotoxin amounts are calculated using the water calibration curve, it turns out that sodium ions disturb activation of the limulus reaction system as the recovery being within a range of -30% to -20%. In contrast, when the endotoxin amounts are calculated using the NaCl calibration curve, it turns out that the measurement can be performed at high accuracy as the recovery being within a range of -10% to +10%.

### [Example 4]

Examining was performed on the influence of deviation between a sodium content of a standard solution and a sodium ion content of a sample to be measured.

Here, a calibration curve to be used for measuring an endotoxin amount with a biochemical luminescence method was prepared by measuring with the biochemical luminescence method using an aqueous NaCl solution with a sodium ion concentration being 140 mmol/L.

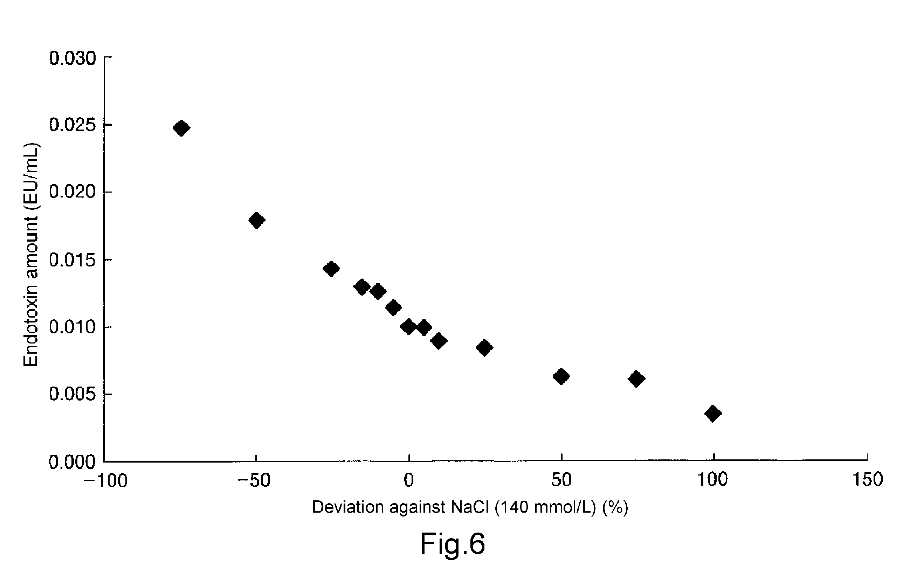

Next, endotoxin-containing solutions of 0.01 EU/L were prepared using aqueous NaCl solutions in which sodium ion concentrations were varied in a range of -75% to +100% taking 140 mmol/L as the basis therefor. Subsequently, measurement was performed with the biochemical luminescence method respectively on the endotoxin-containing solutions of 0.01 EU/L having different sodium ion concentrations, and then, the endotoxin contents were calculated using the abovementioned calibration curve. FIG. 6 shows the results. The horizontal axis of FIG. 6 represents deviation (%) of a sodium ion concentration of a standard solution against a reference value for preparing a calibration curve used by the calculation. The vertical axis thereof represents an endotoxin content calculated using each calibration curve.

According to the results of FIG. 6, it turns out that the recovery being within a range of -30% to +30% can be achieved as long as the sodium ion concentration of the aqueous NaCl solution is within a range of -15% to +25% and that the recovery being within a range of -15% to +15% can be achieved as long as the sodium ion concentration of the aqueous NaCl solution is within a range of -5% to +10%.

As described above, according to the present invention, it is possible to perform measurement at high sensitivity and high accuracy on samples containing sodium ions such as a biological sample (blood and the like), a pharmaceutical product (a dialysis solution and the like), and food. Further, according to the present invention, measurement can be performed at high sensitivity and high accuracy using the same single calibration curve on samples containing sodium (dialysis solutions and the like) and samples containing substantially no sodium ion or less sodium ions such as water (material water for pharmaceutical products and the like).

### EXPLANATIONS OF LETTERS OR NUMERALS

- 1: First container
- 2: Second container
- 10: Quantification kit
- 100: Quantification device

## Claims

1. A quantification method, comprising:
a calibration curve preparing step to measure a standard solution, which has been prepared by adding sodium ions so that a sodium ion content of the standard solution is equaled to a sodium ion content of a sample to be measured with a method employing a reaction that activates a limulus reagent and/or a biochemical luminescent reaction caused by ATP, luciferin, and luciferase, and to prepare a calibration curve that represents a relation between a measurement value and an amount of a component to be measured;
a sample measuring step to measure the sample to be measured with a method being the same as that used in the calibration curve preparing step; and
a quantifying step to find, by using the calibration curve, an amount of the component to be measured in the sample to be measured from a measurement value in the sample measuring step.

2. The quantification method according to claim 1, wherein the component to be measured is endotoxin, beta-glucan, or ATP.

3. A quantification device, performing:
a calibration curve preparing step to measure a standard solution, which has been prepared by adding sodium ions so that a sodium ion content of the standard solution is equaled to a sodium ion content of a sample to be measured with a method employing a reaction that activates a limulus reagent and/or a biochemical luminescent reaction caused by ATP, luciferin, and luciferase, and to prepare a calibration curve that represents a relation between a measurement value and an amount of a component to be measured;
a sample measuring step to measure the sample to be measured with a method being the same as that used in the calibration curve preparing step; and
a quantifying step to find, by using the calibration curve, an amount of the component to be measured in the sample to be measured from a measurement value in the sample measuring step.

4. The quantification device according to claim 3,
wherein the sample to be measured includes a plurality of samples having different sodium ion contents to each other,
the calibration curve is prepared, in the calibration curve preparing step, by performing measurement on the standard solution to which sodium ions are added so that the sodium ion content of the standard solution is equaled to that of a given sample among the plurality of samples, and
in the sample measuring step, measurement is performed without adding sodium ions on the given sample and a sample that has a sodium ion content being equal to or greater than that of the given sample, while measurement is performed on a sample that has a sodium ion content being less than that of the given sample after sodium ions are added so that the sodium ion content of the sample is equaled to that of the given sample.

5. The quantification device according to claim 4, wherein the plurality of samples include a pharmaceutical product and a solution for preparing the pharmaceutical product.

6. The quantification device according to any one of claims 3 to 5, wherein the component to be measured is endotoxin, beta-glucan, or ATP.

7. A quantification kit for quantifying a component to be measured in a sample to be measured with a method employing a reaction that activates a limulus reagent and/or a biochemical luminescent reaction caused by ATP, luciferin, and luciferase or for preparing a calibration curve to be used for the quantifying, the quantification kit comprising a sodium ion source that supplies a predetermined amount of sodium ions to the sample to be measured or a standard solution for preparing the calibration curve.

8. The quantification kit according to claim 7, wherein the sodium ion source supplies sodium ions so that a sodium ion concentration of the sample to be measured or the standard solution for preparing the calibration curve is to be in a range of 135 to 155 mmol/L.
